(19) Europäisches Patentamt

European Patent Office

Office européen des brevets

(11) **EP 1 374 768 A2**

(12) **EUROPEAN PATENT APPLICATION**

(43) Date of publication:
**02.01.2004 Bulletin 2004/01**

(51) Int Cl.⁷: **A61B 5/083**

(21) Application number: **03021573.5**

(22) Date of filing: **23.02.1996**

(84) Designated Contracting States:
**DE FR GB NL**

(30) Priority: **24.02.1995 JP 3669995**
**24.02.1995 JP 3723995**
**24.02.1995 JP 3732495**
**24.02.1995 JP 3736195**
**24.02.1995 JP 3741195**

(62) Document number(s) of the earlier application(s) in accordance with Art. 76 EPC:
**96102772.9 / 0 729 727**

(71) Applicant: **Nihon Kohden Corporation
Tokyo (JP)**

(72) Inventors:
• **Yamamori, Shinji
Tokyo (JP)**

• **Hosaka, Hidehiro
Tokyo (JP)**
• **Ono, Kohei
Tokyo (JP)**
• **Ito, Masami
Tokyo (JP)**
• **Inoue, Masayuki
Tokyo (JP)**
• **Sugiura, Masaki
Tokyo (JP)**

(74) Representative: **Grünecker, Kinkeldey,
Stockmair & Schwanhäusser Anwaltssozietät
Maximilianstrasse 58
80538 München (DE)**

Remarks:
This application was filed on 24 - 09 - 2003 as a divisional application to the application mentioned under INID code 62.

(54) **Capnometer**

(57) The capnometer applies an infrared radiation to a respiratory gas and detects a signal associated with the quantity of transmitted radiation for measuring the concentration of $CO_2$ in the respiratory gas. It comprises a thermal detector for sensing the transmission of infrared radiation, RAM, and control unit that detects a maximum value of the detection signal from the thermal detector during the present inspiration phase, stores the detected maximum value in RAM, performs the first approximation, Kalman filtering or some other suitable method to compute a corrected value that is valid for the time being until a maximum value is detected in the next inspiration phase and determines a density signal by calculating the difference between said corrected value and the detection signal.

*FIG. 1*

EP 1 374 768 A2

**Description**

BACKGROUND OF THE INVENTION

Field of invention

The present invention relates to a capnometer for measuring the concentration of carbon dioxide in expired gases.

Related art

Infrared measurements of carbon dioxide in expired gases are commonly performed with radiation detectors that sense the transmission of radiation associated with the absorption by the carbon dioxide during expiration. The output voltage of the detector is subject to drift for various reasons including the variation in the intensity of radiation from the source and the change in the quantity of radiation due to the contamination of the windows in the sensing part. An apparatus adapted to compensate for such drift is known in the art (see Examined Japanese Patent Publication No. 44614/1985).

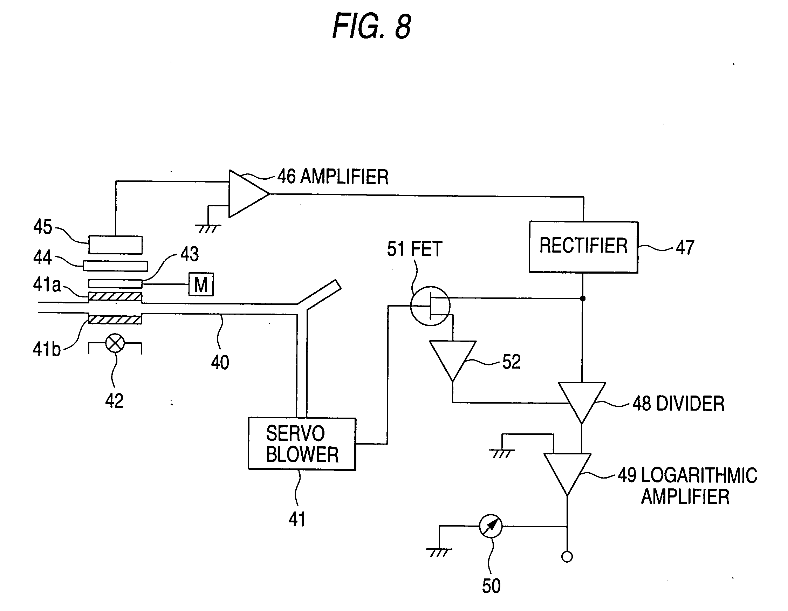

Fig. 8 shows diagrammatically the construction of a capnometer equipped with the known drift compensator. Shown by 40 in Fig. 8 is a tube through which a respiratory gas will pass. One end of the tube 40 is a mouthpiece which is to be inserted into the mouth of a patient and the other end of the tube branches into two parts, one being open to the atmosphere and the other being connected to a servo blower 41 which supplies the patient with air during the inspiration phase. A pair of light-transmitting windows 41a and 41b typically made of sapphire are formed in the middle portion of the tibe 40. A radiation source 42 is provided below the window 41b and a radiation interrupter 43 that has a light-transmitting aperture and which is typically driven to rotate by means of a motor M is provided above the window 41a. Located above the radiation interrupter 43 is a filter 44 that passes only those rays which are absorbed by carbon dioxide and a radiation detector 45 is located above the filter 44. Shown by 46 is an amplifier for amplifying the output voltage of the detector 45; 47 is a rectifier; 48 is a divider; 49 is a logarithmic amplifier; 50 is a recording device; 51 is a field-effect transistor (FET) that conducts during the inspiration phase in response to the output of servo blower 41; and 52 is a memory that stores a voltage corresponding to $CO_2$ concentration of zero during the inspiration phase and which delivers it as an output to the divider 48.
With this arrangement, the radiation from the source 42 passes through the window 41b and the respiratory gas in the tube 40 and emerges from the window 41a to enter the radiation interrupter 43, which chops the incident light at periodic intervals. The chopped rays of light pass through the filter 44 and the transmission of radiation associated with the $CO_2$ concentration is sensed by the detector 45. The output signal from the detector 45 is given as an exponential function, amplified by the amplifier 46 and rectified by the rectifier 47.
The output from the radiation detector 45 contains a drift due, for example, to the change in the quantity of radiation caused by contamination of the filter 44 and windows 41a and 41b and to the variation in the intensity of radiation from the source 42. In order to reject the drift component from the output voltage of the rectifier 47, the servo blower 41 delivers a positive voltage to FET 51 during the inspiration period, whereby the FET conducts and a voltage corresponding to $CO_2$ concentration of zero is stored in the memory 52, from which it is delivered as an output to the divider 48. Upon ending of the inspiration period, the positive voltage from the servo blower 41 disappears and the FET 51 turns off and the output of the rectifier 47 (i.e., a signal associated with the $CO_2$ concentration in the expired gas) is delivered to the divider 48 and divided by the voltage stored in the memory 52 which corresponds to $CO_2$ concentration of zero, whereby the drift component is eliminated. The output of the divider 48 is delivered to the logarithmic amplifier 49 to produce an output signal proportional to the $CO_2$ concentration of the expired gas.
A problem with this capnometer equipped with the prior art drift compensator in a radiation detector is that the detector uses the rather expensive PbSe. This material features fast response but the device temperature will increase upon continued exposure to an infrared radiation and the decreasing resistance will increase the drift. To avoid this problem, the radiation from the source has to be repeatedly chopped at a frequency, say, 200 Hz, higher than the respiration frequency. To meet this need, a radiation interrupter and a drive mechanism such as a motor that drives its rotation have been used to detect the quantity of radiation passing through the respiratory gas. However, this has limited the efforts to reduce the overall size of the system and its power consumption while assuring ruggedness. In addition, the prior art system has had the disadvantage of being costly.

SUMMARY OF THE INVENTION

The present invention has been accomplished under these circumstances and has as an object providing a capnometer that is capable of compensating for the drift in the output voltage of a radiation detector without employing any mechanism for repeatedly chopping the infrared light necessary for the Pbse radiation detector.
This object of the invention can be attained by the capnometer operates on the principle of applying an infrared radiation to a respiratory gas and detecting a signal associated with the quantity of transmitted radiation for measuring the concentration of carbon dioxide in the respiratory gas and which is characterized by comprising:

a thermal detector for sensing the transmission of said infrared radiation;
memory means; and
drift compensating means that detects a maximum value of the detection signal from said thermal detector during the present inspiration phase, stores the detected maximum value in said memory means, computes a corrected value that is valid for the time being until a maximum value is detected during the next inspiration phase, and that determines a density signal by calculating the difference between said corrected value and the detection signal.

In the capnometer of the present invention, said drift compensating means detects a maximum value of the detection signal from said thermal detector during the present inspiration phase, stores the detected maximum value in said memory means, and determines a density signal by computing the difference between said stored maximum value and a detection signal delivered subsequent to the point of detection of said maximum value.

In the capnometer of the present invention, said drift compensating means detects a maximum value of the detection signal from said thermal detector during the present inspiration phase, stores the detected maximum value in said memory means, computes a straight line connecting the maximum values for the present and preceding inspiration phases, determines a correction line extending to the point of detection of a maximum value during the next inspiration phase, a density signal by calculating the difference between said correction line and a detection signal delivered subsequent to the point of detection of the present maximum value.

In the capnometer of the present invention, said drift compensating means detects a maximum value of the detection signal from said thermal detector during the present inspiration phase, stores the detected maximum value in said memory means, updates the stored maximum value if it is exceeded by any detection signal that is delivered from said thermal detector before the detection of a maximum value during the subsequent inspiration phase, stores the updated maximum value, reads the stored maximum values successively out of the memory means, subjects them to Kalman filtering so as to output a corrected value, and determines a density signal by calculating the difference between said corrected value and the detection signal.

Accroding to the present invention, the thermal detector delivers a detection signal associated with the $CO_2$ concentration of the respiratory gas to be analyzed and a maximum value of the detection signal during the present inspiration phase is stored in the memory means. Then, a corrected value valid for the time being until a maximum value is detected during the next inspiration phase is computed and the difference between the corrected value and the detection signal is calculated to determine a density signal. The desired $CO_2$ concentration is determined on the basis of said density signal.

According to the present invention, the thermal detector delivers a detection signal associated with the $CO_2$ concentration of the respiratory gas to be analyzed and a maximum value of the detection signal during the present inspiration phase is stored in the memory means. Then, the difference between the stored maximum value and a detection signal delivered subsequent to the point of detection of said maximum value is computed to determine a density signal. The desired $CO_2$ concentration is determined on the basis of said density signal.

According to the present invention, the thermal detector delivers a detection signal associated with the $CO_2$ concentration of the respiratory gas to be analyzed and a maximum value of the detection signal during the present inspiration phase is stored in the memory means. Then, a straight line connecting the maximum values detected during the present and preceding inspiration phases is determined and extended to the point of detection of a maximum value during the next inspiration phase to provide a correction line. Subsequently, the difference between this correction line and a detection signal delivered subsequent to the point of detection of a maximum value during the present inspiration phase is calculated to determine a density signal. The desired $CO_2$ concentration is determined on the basis of said density signal.

According to the present invention, the thermal detector delivers a detection signal associated with the $CO_2$ concentration of the respiratory gas to be analyzed and a maximum value of the detection signal during the present inspiration phase is stored in the memory means. If the stored maximum value is exceeded by any detection signal that is delivered from the thermal detector before the detection of a maximum value during the subsequent inspiration phase, the stored maximum value is updated by replacement with said greater detection signal, which is stored in the memory means. Then, the stored maximum values are successively read out of the memory means and subjected to Kalman filtering so as to output a corrected value. The difference between this corrected value and the detection signal is calculated to determine a density signal. The desired $CO_2$ concentration is determined on the basis of this density signal.

## BRIEF DESCRIPTION OF THE DRAWINGS

Fig. 1 is a block diagram showing the composition of the capnometer of the invention;
Fig. 2 is a diagram illustrating the operating theory of the invention by means of correction lines for the detection signal from a thermal detector;

Fig. 3 is a diagram also illustrating the operating theory of the invention by means of correction lines for the detection signal from the thermal detector;

Fig. 4 is a flowchart describing the sequence of processing steps in the embodiment shown in Fig. 3;

Fig. 5 illustrates the operating theory of the invention by means of correction lines constructed by the Kalman filtering of the detection signal from the thermal detector;

Fig. 6 is a flowchart describing the sequence of processing steps in the embodiment shown in Fig. 5;

Fig. 7 is a timing chart showing the waveforms obtained by measuring the $CO_2$ concentration in the embodiments; and

Fig. 8 shows diagrammatically the composition of a capnometer equipped with a prior art drift compensator.

## DETAILED DESCRIPTION OF THE PREFERRED EMBODIMENTS

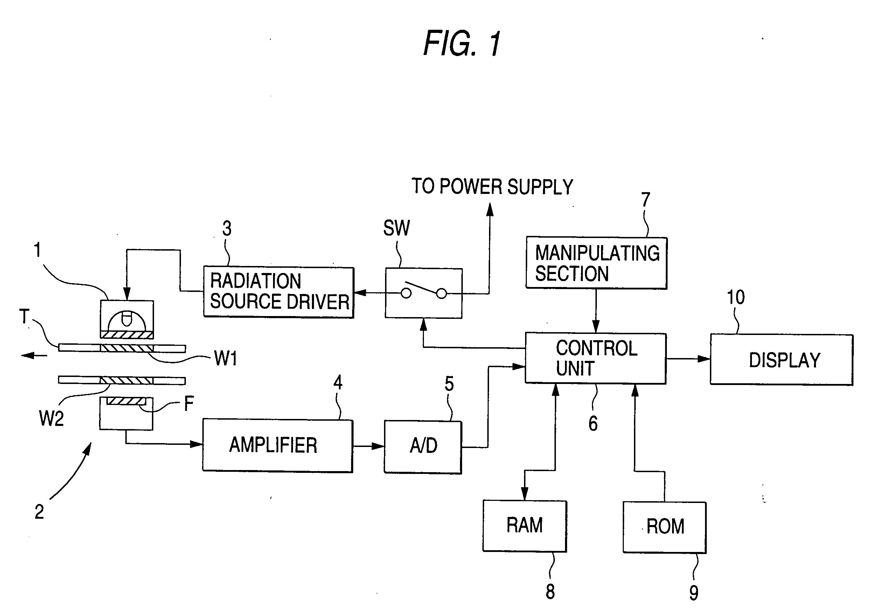

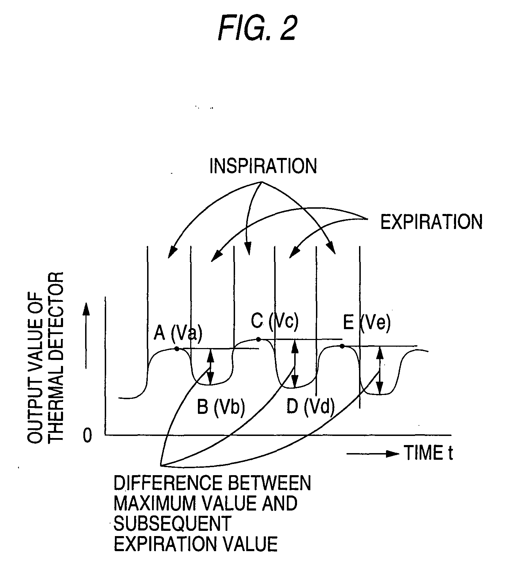

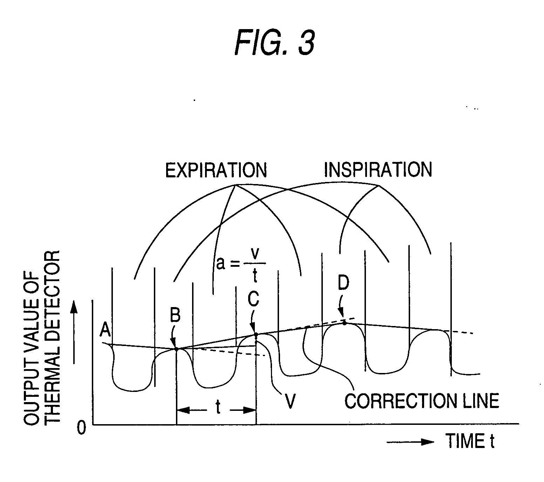

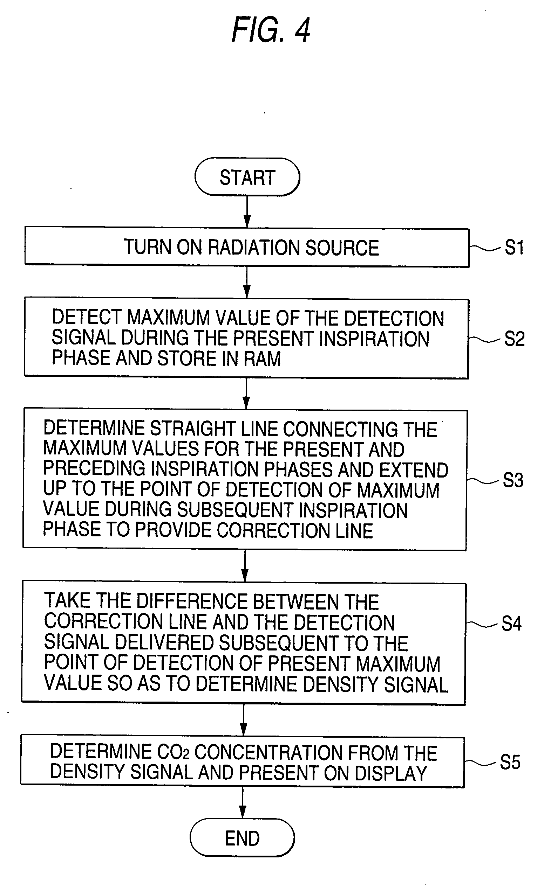

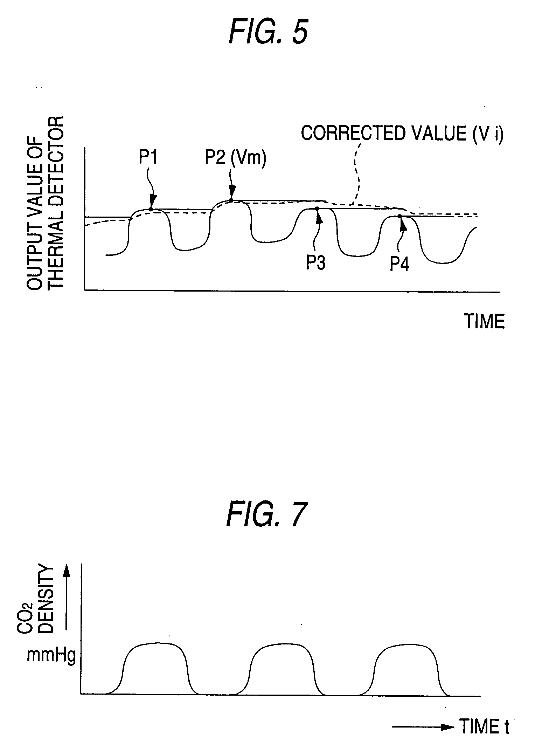

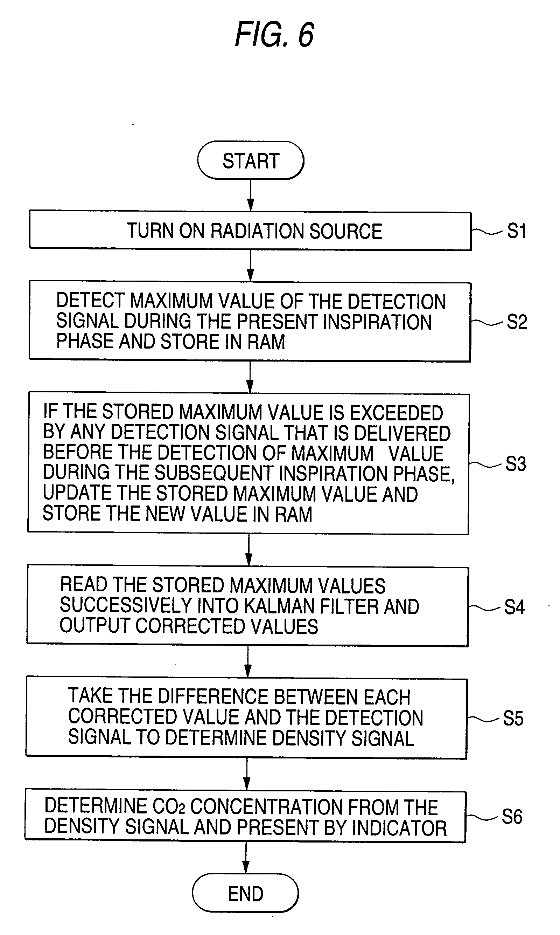

Embodiments of the capnometer of the invention will now be described with reference to the accompanying drawings, in which Fig. 1 is a block diagram showing the composition of the capnometer of the invention; Figs. 2 and 3 illustrate the operating theory of the invention by means of correction lines for the detection signal from a thermal detector; Fig. 4 is a flowchart describing the sequence of processing steps in the embodiment shown in Fig. 3; Fig. 5 illustrates the operating theory of the invention by means of correction lines constructed by the Kalman filtering of the detection signal from the thermal detector; Fig. 6 is a flowchart describing the sequence of processing steps in the embodiment shown in Fig. 5; Fig. 7 is a timing chart showing the waveforms obtained by measuring the $CO_2$ concentration in the embodiments; and Fig. 8 shows diagrammatically the composition of a capnometer equipped with a prior art drift compensator.

Before going into details of the embodiments, we first describe the operating theory of the invention. The invention uses a thermopile as a thermal detector for sensing the intensity of an infrared radiation as it varies with the concentration of $CO_2$ in an expired gas. Thermopiles (e.g. Model S60 of Dexter Research Center, USA) are subject to a smaller amount of drift than the conventional PbSe and they are less expensive. On the other hand, thermopiles have unique properties and must be used in compliance with such characteristics. First of all, response speeds faster than 5 ms are said to be necessary for accomplishing precise sampling with capnometers; however, thermopiles are so slow in response (50 - 200 ms) that it is difficult to meet this requirement for response.

Another problem with the capnometer using a thermopile is that the detection signal can potentially contain drift components due to various factors such as the change in the quantity of infrared rays from the radiation source, the clouding or contamination of the windows in the expired gas sensing portion and the structure of the thermopile per se. The drift component due to the structure of the thermopile need be compensated since it occurs on account of the change in the temperature of the environment in which the capnometer is used. Stated more specifically, the thermopile has many couple of hot and cold junction and due to the time constant mismatch between the two junctions, a drift will appear in the detection signal. The hot junction having a small heat capacity will respond rapidly to an abrupt change in the ambient temperature; however, the cold junctions which make thermal contact with the vessel or heat sink have a large heat capacity and do not respond as fast as the hot junction. As a result, the detection signal which is delivered in accordance with the temperature difference between the hot and cold junctions will contain an undesired drift component until the cold junctions achieve thermal equilibrium with the ambient temperature.

It is therefore an object of the invention to provide a capnometer that is adapted to compensate for any drift that occurs in the detection signal due to the structure of the thermopile when there is an abrupt change in the temperature or the environment in which the system is used.

A specific procedure for drift compensation comprises sensing a maximum value of the detection signal from the thermopile in each inspiration phase, storing the sensed maximum value, detecting an output value in each subsequent expiration phase and calculating the difference between the stored maximum value and the output value for the subsequent expiration phase so as to determine a density signal.

Referring now to Fig. 1, symbol T designates an air way adapter through which both the expired and inspired gas will pass. Windows W1 and W2 each made of a transparent material such as a plastic sheet are formed in opposed areas of the air way adapter. One end portion of air way adapter T (which is on the left of Fig. 1) serves as a mouthpiece which is to be inserted into the mouth of the patient and the other end portion (which is on the right of Fig. 1) is open to the atmosphere. Both windows W1 and W2 are protected against clouding such as with the water vapor in the expired gas. An infrared radiation source 1 such as an infrared lamp is provided just above the widow W1 such that an infrared radiation is applied through the window W1. A thermal detector 2 in the form of the above-described thermopile is provided just below the window W2 such that the infrared radiation supplied from the source 1 through the windows W1 and W2 is detected. A filter F is located in the light-receiving surface of the thermal detector 2 and it selectively passes those rays which have such a wavelength (about 4.3 $\mu$m) that they are absorbed by carbon dioxide in the expired gas.

Shown by 3 is a radiation source drive unit that is typically composed of a constant-current circuit and which allows the source 1 to emit an infrared radiation of a constant intensity. The drive unit 3 is connected to a switch SW which is operated by a control unit (to be described later) to turn the radiation source 1 on and off as required. Switch SW is typically composed of an electronic switch such as a transistor and normally on. Shown by 4 is an amplifier for amplifying the detection voltage of the thermal detector 2; 5 is an analog-digital converter for converting the output of amplifier 4 to a digital signal. Control unit 6 is typically composed of a CPU and controls the overall system in accordance with a $CO_2$ measuring control program stored in ROM 9 which will be described later.

Shown by 7 is a manipulating section typically composed of a plurality of buttons which are to be touched for various purposes such as turning the radiation source 1 on and off in a trial operation and providing settings of the necessary data.

Shown by 8 is a RAM that temporarily stores and holds various data including the parameter settings and the measured $CO_2$ concentrations; 9 is a ROM which contains a control program for performing automatic $CO_2$ concentration measurements by compensating for the drift in the voltage output from the thermal detector 2 in accordance with the above-described operating theory of the present invention.

First embodiment

Fig. 2 illustrates how drift compensation is performed in a first embodiment of the invention. Symbols A, C and E refer to the points at which maxima in the detection signal from the thermopile were sensed during the inspiration phase, namely, for the inhaled gas substantially free from $CO_2$. Symbols B and D refer to the points at which signals were detected during expiration phases subsequent to points A and C, respectively, said signals having been reduced in accordance with the concentration of $CO_2$ in the exhaled gas. Consider, for example, point A. According to the invention, a maximum value Va is detected at point A during the inspiration phase and stored; then, an output value Vb is detected at point B during the subsequent expiration phase and subtracted from Va; this procedure is repeated for each cycle of inspiration and expiration phases. This embodiment, in which a maximum value for each inspiration phase is stored such that the difference from an output value for the subsequent expiration phase is determined, is effective in the case where the drift to be compensated is mild or small.

However, it is not very effective in compensating for a large drift such that the maximum value of detection signal varies greatly from one inspiration phase to another. Referring again to Fig. 2, the maximum value Vc may be held up to point E, where it is replaced by a maximum value that is detected in the next inspiration phase. In the drift is as large as in this illustrated case, the detection signal involves substantial discontinuities that are great enough to reduce the precision in $CO_2$ measurements.

Second embodiment

Fig. 3 is a timing chart showing the waveforms obtained in the compensation for the drift in the output of the thermal detector using correction lines in accordance with a second embodiment of the invention. In Fig. 3, symbols A - D represent the points at which maxima are sensed for the detection signal from the thermopile during successive inspiration phases. The compensating procedure typically goes like this: if B is supposed to be the point at which a maximum value is sensed by the thermopile during the present inspiration phase, point A at which a maximum value was sensed by the thermopile during the preceding inspiration phase is connected to point B by a straight (solid) line and the line is extended up to point C where a maximum value is sensed during the subsequent inspiration phase (the extended line is dashed and referred to as a "correction line"); similarly, if C is supposed to be the point at which a maximum value is sensed by the thermopile during the present inspiration phase, point B at which a maximum value was sensed during the preceding inspiration phase is connected to point C by a straight line and the line is extended up to point D where a maximum value is sensed during the subsequent inspiration phase (the extended line is also dashed and referred to as "correction line"). Output values from the thermopile are sensed for the expiration phases subsequent to the respective points of maxima sensing and subtracted from the associated correction lines to determine density signals, each of which is used as a basis for computing the $CO_2$ concentration.

The straight line connecting two points of maxima detection in successive inspiration phases may be provided by a stored equation for straight line $v = at,$ where $v$ is the difference between maxima at the two points, $t$ is the time difference between the two points, and $\underline{a}$ is the slope of the line. Referring to Fig. 3, the straight (solid) line connecting two points, say, B and C, may be determined on the basis of the detected maxima, which are stored and processed by the equation $v = at$, which the horizontal distance between the maxima being taken as the time difference t between points B and C, the vertical distance between the maxima being taken as v, and the ratio of $v$ to $t$ $(v/t)$ being taken as the slope $\underline{a}$.

According to this embodiment, a degree of the discontinuities applied to the detection signal becomes small as compared with the first embodiment such that the measurments on the basis of the difference between the maximum values during inspiration when the drift appears in an one way direction (in this embodiment, upper direction) such as points B - C - D.

The operation of the capnometer having this structure will now be described with reference to the flowchart shown in Fig. 4. At the commencement of measurement, the power switch (not shown) turns on the radiation source 1 (step S1). Thereafter, the patient is allowed to breathe so that his respiratory gas goes into and comes out of the air way adapter T via the mouthpiece inserted into his mouth.

The transmission of the radiation which varies with the change in the $CO_2$ concentration of the respiratory gas is received by the thermal detector 2 in such a way that the point at which the detector 2 delivers an increased output is recognized as an inspiration and a maximum value corresponding to $CO_2$ concentration of zero is picked up from the detection signal from the thermal detector 2 during the inspiration period and stored in RAM (step S2). The desired maximum value can be identified by performing a suitable processing on the detection signal from the thermal detector 2, for example, by calculating the differential for successive data on the time base.

The maximum output value for the present inspiration phase that has been picked up by the thermal detector 2 in step S2 is connected by a straight line to the maximum value for the preceding inspiration phase on the basis of the foregoing operating theory behind the first embodiment of the invention and the connecting line is extended as a correction line up to the point where a maximum value for the subsequent inspiration phase is detected (step S3).

Then, a lower detection signal delivered from the thermal detector 2 at a time subsequent to the detection of the present maximum value is recognized as representing an expiration and the output value for the expiration phase is detected and the difference from the correction line determined in step S3 is calculated to determine a density signal (step S4).

With the density signal being thusly determined, the $CO_2$ concentration is determined and displayed on the display unit 10 (step S5). If the display unit 10 is a bar graph, varying lengths of bars will appear as the $CO_2$ concentration changes as shown in Fig. 7.

Thus, in the second embodiment of the invention, the correction line is extended up to the point of detection of a maximum value for each subsequent inspiration phase and used to determine the density signal. Even if the detection signal from the thermal detector contains a large drift component that produces a discontinuity between two successive expiration phases for the determination of a density signal, satisfactory drift compensation can be accomplished by the method of this embodiment.

Third embodiment

A third embodiment of the invention will now be described with reference to the case where drift compensation is performed by Kalman filtering which is known as one of the computer techniques used to give a best estimate for a given system. Kalman filtering is also known to be capable of processing on a real-time basis and, hence, providing good accommodation of the latest data.

Fig. 5 shows the waveforms of the corrected values that are delivered from a Kalman filter and on the basis of such corrected values, the output from the thermopile is drift compensated in the third embodiment. Referring to Fig. 5, P1 - P4 designate maxima that are detected from the thermopile during successive inspiration phases and Vm represents a maximum value for the inspiration phase that is picked up from the detection signal at point P2. As shown, Vm is on the solid line. On the other hand, the dashed line indicates a corrected value VI which is delivered as a best estimate from the Kalman filter and which can be computed by the following equation, with the maximum value Vm being entered as an input parameter:

$$VI(n+1) - VI(n) + (Vm - VI(n))/B(n+1) \qquad (1)$$

where the function B(n+1) is expressed by:

$$B(n+1) = (1 + \alpha \cdot B(n))$$

where $\alpha$ is a predetermined Kalman coefficient which affects the correcting characteristics of the filter; n is the sampling interval taking discrete values such as 0, 1, 2, ...; VI(n+1) is the filter output at the present time; VI(n) is the filter output at the preceding point of time; and Vm is a maximum value of the detection signal stored at the present time.

The initial values of VI and B are VI(0) = 0 and B(1) = 1.

Thus, corrected value VI(n+1) can be obtained by substituting the present maximum value for the thermopile into equation (1).

As shown by the dashed line in Fig. 5, the corrected value VI(n+1) closely follows and approaches the maxima that are detected in successive inspiration phases for entry into the computer. Hence, by determining the difference between this corrected value and the detection signal from the thermopile, one can obtain a density signal corresponding to the $CO_2$ concentration to be measured.

The operation of the capnometer having the structure described above will now be described with reference to the flowchart shown in Fig. 6. At the commencement of measurement, the power switch (not shown) turns on the radiation source 1 (step S1). Thereafter, the patient is allowed to breathe so that his respiratory gas goes into or comes out of the air way adapter T via the mouthpiece inserted into his mouth.

The transmission of the infrared radiation which varies with the change in the $CO_2$ concentration of the respiratory gas is received by the thermal detector 2 in such a way that the point at which the detector delivers an increased output is recognized as an inspiration and a maximum value for the present inspiration phase is picked up from the detection signal from the detector 2 and stored in RAM (step S2). The desired maximum value can be identified by performing a suitable processing on the detection signal from the thermal detector 2, for example, by calculating the differential from successive data on the time base.

If, prior to the detection of a maximum value for the subsequent inspiration phase, the detection signal from the thermal detector 2 exceeds the maximum value stored in RAM 8, the latter is updated and the new value is stored in RAM 8 (step S3).

The control unit 6 successively reads the stored maxima out of RAM 8 and performs Kalman filtering on the stored data in accordance with the above-described operating theory of the third embodiment of the invention, whereby corrected values are successively delivered (step S4).

Then, the difference between each of the corrected values and the detection signal from the thermal detector 2 is calculated to determine a density signal that (step S5).

The control unit 6 determines the $CO_2$ concentration on the basis of the density signal and sends the $CO_2$ concentration to the indicator unit 10, which provides an optical and/or audible indication of the $CO_2$ concentration (step S6). An exemplary presentation of the $CO_2$ concentration profile is given in Fig. 7. If the indicator unit 10 is adapted to construct a bar graph as in the prior art, varying lengths of bars will appear as the concentration of $CO_2$ changes.

Thus, in the third embodiment of the invention, corrected values are determined by Kalman filtering and drift compensation is accomplished by taking the difference between each corrected value and the associated detection signal. As a result, there is provided a capnometer capable of drift compensation in such a way that the compensated signal will not contain any point of discontinuity despite abrupt changes in the ambient temperature.

As will be understood from the foregoing description, the use of a thermal detector as an infrared radiation detector in the capnometer of the invention eliminates the need to employ mechanical parts, such as a chopper (radiation interrupter) and a motor for driving it to rotate, that have been necessary in the conventional radiation detectors. This offers the advantage of providing ease in reducing the overall size of the system while increasing its ruggedness and reducing the production cost.

Another feature of the capnometer of the invention is that the drift that occurs in the detection signal from the thermal detector in response to an abrupt change in the ambient temperature can be corrected by Kalman filtering and this offers the advantage of achieving $CO_2$ concentration measurements to provide a consistent profile having no points of discontinuity.

**Claims**

1. A capnometer comprising:

   a light source (1 ) for applying an infrared radiation to a respiratory gas;
   a thermal detector (2) for sensing the transmission of said infrared radiation and for generating a signal associated with the quantity of transmitted radiation,
   memory means (8,9) for storing said signal generated from said thermal detector, and
   drift compensating means (6) for detecting a maximum value of the detection signal from said thermal detector during the present inspiration phase, for storing the detected maximum value in said memory means, for updating the stored maximum value if it is exceeded by any detection signal that is delivered from said thermal detector before the detection of a maximum value during the subsequent inspiration phase, for storing the updated maximum value, for reading the stored maximum values successively out of the memory means, for subjecting them to Kalman filtering so as to output a corrected value, and for determining a density signal by calculating the difference between said corrected value and the detection signal during the subsequent expiration.

2. A capnometer according to claim 1, wherein said thermal detector includes a thermopile.

3. A capnometer according to claim 1, wherein said thermal detector (2) senses the transmission of said infrared radiation without chopping and generates a signal associated with the quantity of transmitted radiation.

*FIG. 1*

# FIG. 2

# FIG. 3

# FIG. 4

```
        ┌──────────────┐
        │    START     │
        └──────┬───────┘
               │
               ▼
   ┌──────────────────────────────────┐
   │  TURN ON RADIATION SOURCE        │──── S1
   └──────────────┬───────────────────┘
                  │
                  ▼
   ┌──────────────────────────────────┐
   │  DETECT MAXIMUM VALUE OF THE      │
   │  DETECTION SIGNAL DURING THE      │──── S2
   │  PRESENT INSPIRATION PHASE AND    │
   │  STORE IN RAM                     │
   └──────────────┬───────────────────┘
                  │
                  ▼
   ┌──────────────────────────────────┐
   │  DETERMINE STRAIGHT LINE          │
   │  CONNECTING THE MAXIMUM VALUES    │
   │  FOR THE PRESENT AND PRECEDING    │
   │  INSPIRATION PHASES AND EXTEND    │──── S3
   │  UP TO THE POINT OF DETECTION OF  │
   │  MAXIMUM VALUE DURING SUBSEQUENT  │
   │  INSPIRATION PHASE TO PROVIDE     │
   │  CORRECTION LINE                  │
   └──────────────┬───────────────────┘
                  │
                  ▼
   ┌──────────────────────────────────┐
   │  TAKE THE DIFFERENCE BETWEEN THE  │
   │  CORRECTION LINE AND THE          │
   │  DETECTION SIGNAL DELIVERED       │
   │  SUBSEQUENT TO THE POINT OF       │──── S4
   │  DETECTION OF PRESENT MAXIMUM     │
   │  VALUE SO AS TO DETERMINE         │
   │  DENSITY SIGNAL                   │
   └──────────────┬───────────────────┘
                  │
                  ▼
   ┌──────────────────────────────────┐
   │  DETERMINE CO2 CONCENTRATION FROM │
   │  THE DENSITY SIGNAL AND PRESENT   │──── S5
   │  ON DISPLAY                       │
   └──────────────┬───────────────────┘
                  │
                  ▼
        ┌──────────────┐
        │     END      │
        └──────────────┘
```

S2: DETECT MAXIMUM VALUE OF THE DETECTION SIGNAL DURING THE PRESENT INSPIRATION PHASE AND STORE IN RAM

S3: DETERMINE STRAIGHT LINE CONNECTING THE MAXIMUM VALUES FOR THE PRESENT AND PRECEDING INSPIRATION PHASES AND EXTEND UP TO THE POINT OF DETECTION OF MAXIMUM VALUE DURING SUBSEQUENT INSPIRATION PHASE TO PROVIDE CORRECTION LINE

S4: TAKE THE DIFFERENCE BETWEEN THE CORRECTION LINE AND THE DETECTION SIGNAL DELIVERED SUBSEQUENT TO THE POINT OF DETECTION OF PRESENT MAXIMUM VALUE SO AS TO DETERMINE DENSITY SIGNAL

S5: DETERMINE $CO_2$ CONCENTRATION FROM THE DENSITY SIGNAL AND PRESENT ON DISPLAY

## FIG. 5

## FIG. 7

# FIG. 6

START

TURN ON RADIATION SOURCE — S1

DETECT MAXIMUM VALUE OF THE DETECTION SIGNAL DURING THE PRESENT INSPIRATION PHASE AND STORE IN RAM — S2

IF THE STORED MAXIMUM VALUE IS EXCEEDED BY ANY DETECTION SIGNAL THAT IS DELIVERED BEFORE THE DETECTION OF MAXIMUM VALUE DURING THE SUBSEQUENT INSPIRATION PHASE, UPDATE THE STORED MAXIMUM VALUE AND STORE THE NEW VALUE IN RAM — S3

READ THE STORED MAXIMUM VALUES SUCCESSIVELY INTO KALMAN FILTER AND OUTPUT CORRECTED VALUES — S4

TAKE THE DIFFERENCE BETWEEN EACH CORRECTED VALUE AND THE DETECTION SIGNAL TO DETERMINE DENSITY SIGNAL — S5

DETERMINE $CO_2$ CONCENTRATION FROM THE DENSITY SIGNAL AND PRESENT BY INDICATOR — S6

END

# FIG. 8

EP 1 374 768 A2